# FASCICULE DE BREVET EUROPEEN

(11) **EP 2 719 423 B1**
(45) Date de publication et mention de la délivrance du brevet: **18.02.2015**
(21) Numéro de dépôt: 13169172.7
(22) Date de dépôt: 24.05.2013
(51) Int. Cl.: A61N 1/05

(54) **Microsonde atraumatique de détection/stimulation**
Atraumatische Mikrosonde zur Erfassung/Stimulation
Atraumatic detection/stimulation microprobe

(30) Priorité: 12.10.2012 FR 1259759
(43) Date de publication de la demande: 16.04.2014
(73) Titulaire: Sorin CRM SAS, 92140 Clamart Cedex (FR)
(72) Inventeur: Shan, Nicholas, 91260 Juvisy sur Orge (FR); Régnier, Willy, 91160 Longjumeau (FR)
(74) Mandataire: Dupuis-Latour, Dominique

(56) Documents cités:
- EP-A1- 2 455 131
- FR-A1- 2 550 454
- US-A- 4 934 049

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boitier généralement désigné "générateur", raccordé électriquement et mécaniquement à une ou plusieurs "sondes" intracorporelles munie(s) d'électrodes destinées à venir en contact avec les tissus sur lesquels on souhaite appliquer des impulsions de stimulation et/ou recueillir un signal électrique : myocarde, nerf, muscle...

La présente invention concerne plus précisément une microsonde de détection/stimulation destinée à être implantée dans des réseaux veineux, artériels ou lymphatiques.

Le principe actuel de stimulation électrique utilise un dispositif, appelé généralement "sonde", qui est un objet implanté au travers de divers vaisseaux, veineux, artériels ou lymphatiques, dont la fonction est de transmettre un signal électrique au tissu-cible tout en garantissant les propriétés générales suivantes :
- facilité d'implantation par le médecin dans un réseau de vaisseaux du patient, et en particulier facilité : à faire progresser la sonde dans les vaisseaux par poussée, à faire suivre à la sonde des trajets tortueux et passer des embranchements, et à transmettre des couples ;
- visibilité aux rayons X afin de permettre au médecin une navigation aisée à travers les vaisseaux du réseau sous radioscopie X ;
- atraumaticité de la sonde au niveau des vaisseaux, ce qui nécessite une grande souplesse de structure et l'absence de transition rigide ou d'angle vif ;
- aptitude à transmettre un signal électrique aux tissus et à faire des mesures électriques monopolaires ou multipolaires de manière stable ;
- biocompatibilité avec les tissus vivants pour permettre une implantation à long terme ;
- biostabilité, en particulier résistance à la corrosion dans le milieu vivant et résistance à la sollicitation mécanique en fatigue liée aux mouvements du patient et des organes ;
- aptitude à la stérilisation (rayons gamma, température ...) sans subir de dommages ; et
- compatibilité avec l'imagerie IRM, particulièrement importante en neurologie.

L'architecture actuelle des sondes connues répondant à ces besoins peut se résumer à une structure généralement creuse permettant le passage d'un mandrin ou d'un fil-guide, et comprenant des composants de type câbles conducteurs isolés, reliés à des électrodes mécaniques pour assurer la conductivité électrique, la radio-opacité, etc.

Il s'agit donc de sondes nécessitant l'assemblage complexe d'un nombre important de pièces, de fils et d'isolants associés, créant des risques non négligeables de rupture compte tenu des contraintes mécaniques à long terme auxquelles elles sont exposées.

Des exemples de telles sondes sont donnés dans les US 6 192 280 A et US 7 047 082 A.

Parmi les difficultés rencontrées, on peut citer la gestion des gradients de raideur liés aux pièces mécaniques utilisées, qui affectent fortement les propriétés d'implantabilité et de résistance mécanique sur le long terme (fatigue). D'autres difficultés peuvent également se poser en termes de fatigue des assemblages. En effet, toute zone de transition de rigidité est susceptible d'induire des risques de fatigue, des difficultés à stériliser du fait de la présence de zones d'accès difficile, et des problèmes de tenue des jonctions de conducteurs au niveau de la liaison avec les électrodes et le connecteur.

Par ailleurs, la tendance clinique dans le domaine des sondes implantables est d'en réduire la taille afin de les rendre moins invasives et plus faciles à manipuler à travers les vaisseaux.

La taille actuelle des sondes implantables est typiquement de l'ordre de 4 à 6 French (1,33 à 2 mm). Cependant, il est clair que la réduction de la taille des sondes augmenterait leur complexité et imposerait des contraintes techniques génératrices de risques.

Pourtant, une telle réduction, à moins de 1,5 French (0,5 mm), voire 1 French (0,33 mm) par exemple, ouvrirait des perspectives d'applications médicales dans des domaines variés allant de la cardiologie à la neurologie en présence d'un réseau veineux, artériel ou même lymphatique, comme le réseau veineux cérébral ou le réseau veineux du sinus coronaire.

Aujourd'hui, la technologie de stimulation électrique a permis des avancées importantes dans le domaine de la neuromodulation, qui consiste à stimuler des zones-cibles du cerveau pour le traitement de la maladie de Parkinson, l'épilepsie et autres maladies neurologiques. On pourrait donc imaginer parvenir avec ce type de technologie à traiter de nouvelles régions difficilement atteignables aujourd'hui, ceci au moyen de sondes de stimulation de petite taille, ou "microsondes", présentant une grande robustesse afin de garantir la biostabilité à long terme. Avec des microsondes de taille réduite, il est possible d'envisager notamment le passage dans des vaisseaux coronaires profonds pour réaliser par exemple un dispositif de stimulation du ventricule gauche via deux zones distinctes. Une telle technique permettrait également une approche moins invasive de ces traitements et surtout une efficacité supérieure des traitements administrés.

Il serait en outre possible de connecter une ou plusieurs microsondes à travers le réseau de vaisseaux considéré jusqu'à leur localisation-cible. Leur mise en place pourrait être effectuée, du fait de leur taille réduite, par des dispositifs de guidage utilisés aujourd'hui en neuroradiologie interventionnelle pour la libération de ressorts (coils) lors du traitement des anévrismes intracrâniens. En particulier, des microsondes d'au plus 1,5 French permettraient d'utiliser des cathéters implantables de diamètre interne de 1,6 French.

S'agissant plus particulièrement de la resynchronisation du rythme cardiaque, les sondes gauches actuellement utilisées sont placées dans, ou juste à l'entrée du sinus coronaire car la progression y est difficile et souvent limitée par la réduction progressive du diamètre de passage du sinus. Des microsondes monopolaires de section inférieure à 1 French ou multipolaires dont le diamètre des microcâbles reste également inférieur à 1 French ouvrent de nouvelles possibilités aux médecins pour envisager de dépasser le sinus coronaire et pouvoir positionner les électrodes de stimulation dans les vaisseaux coronaires profonds du coeur gauche.

Le EP 2 455 131 A1 (Sorin CRM SAS) décrit une sonde constituée dans sa partie active, distale, par un microcâble présentant un diamètre de l'ordre de 0,5 à 2 French (0,17 à 0,66 mm). Ce microcâble comprend un câble de coeur électriquement conducteur formé par un toron d'une pluralité de brins composites, avec une couche d'isolement en polymère entourant partiellement le câble de coeur et dénudée ponctuellement de façon à mettre à nu le microcâble en un ou plusieurs points constituant ensemble un réseau d'électrodes reliées en série, l'extrémité libre du toron étant par ailleurs dotée d'une électrode distale rapportée. Cette configuration permet de multiplier les points de stimulation dans une zone profonde du réseau coronarien.

Cependant, cela entraine de nouveaux problèmes techniques. En particulier, les veines du réseau coronaire ont une section très étroite, et donc une épaisseur très faible, induisant ainsi un risque de perforation par les microsondes ayant une structure de type microcâble.

De plus, lors de sa fabrication, diverses irrégularités (arêtes blessantes, bouts pointus, brins hors de l'isodiamètre, etc.) apparaissent là où le microcâble est coupé (au moyen d'une pince coupante ou par tir laser) au niveau de l'extrémité distale des brins, irrégularités qui sont autant de sources potentielles d'endommagement des parois veineuses.

Ces sondes doivent donc présenter une extrémité distale la moins traumatique possible pour les veines lors de la pose et pendant la vie du patient.

De plus, les mouvements cardiaques (diastole, systole) entrainent des contraintes soit par frottement, soit par compression de la sonde sur les parois veineuses. Le risque induit est une perforation de la veine et/ou du sac péricardique provoquant une hémorragie plus ou moins grave pour le patient.

Aussi, le but de la présente invention est de proposer une microsonde ayant une structure de type microcâble qui serait en conformité avec les propriétés générales des sondes implantables telles qu'elles ont été exposées plus haut, et plus spécialement la propriété d'atraumaticité, par un aménagement spécifique de l'extrémité distale de la microsonde permettant d'éviter les irrégularités risquant de blesser les parois des veines.

De même, la microsonde de l'invention doit pouvoir répondre à un certain nombre d'exigences.

Au niveau de la fiabilité, il s'agit d'assurer la souplesse de l'extrémité du microcâble en utilisant des matériaux biocompatibles et très souples, tout en garantissant les fonctions de stimulation électrique et d'étanchéité. Il convient également de prévenir le risque de corrosion du microcâble en fonction des matériaux utilisés.

Au niveau des performances, il s'agit de :
- maintenir l'extrémité distale du microcâble dans un diamètre extérieur typique hors-tout de 1,5 French (0,50 mm), pour garantir le passage dans des vaisseaux coronaires profonds et dans le cathéter, et assurer un profil isodiamétrique ;
- ne pas altérer la grande flexibilité de l'extrémité distale de la microsonde afin de conserver une maniabilité maximale, notamment au passage à travers les petites veines ; et
- faciliter et simplifier la procédure en maitrisant la progression dans le système nerveux de l'assemblage microsonde/cathéter.

Dans un autre contexte, le FR 2 550 454 A1 décrit un cathéter creux pour l'injection de produits de contraste. L'extrémité libre ouverte du cathéter présente un rebord périphérique muni d'un ballonnet annulaire gonflable ou autrement déformable, pour éviter un contact trop brusque entre l'extrémité de la gaine flexible du cathéter et les tissus de l'organe où est introduit ce cathéter.

L'invention est définie dans les revendications qui suivent.

Conformément à l'invention, les buts précités sont atteints grâce à une microsonde de détection/stimulation destinée à être implantée dans des réseaux veineux, artériels ou lymphatiques, et comportant au moins un microcâble de diamètre hors-tout au plus égal à 1,5 French (0,50 mm) comprenant un câble de coeur électriquement conducteur, formé par au moins un toron d'une pluralité de brins métalliques élémentaires.

Cette microsonde est remarquable en ce que le câble de coeur présente une extrémité distale munie d'un dispositif de protection atraumatique comprenant un moyen d'enrobage des extrémités distales des brins élémentaires du câble de coeur, et un embout de protection en matériau à la fois déformable et incompressible, enveloppant le moyen d'enrobage. L'invention s'adresse aussi bien à une sonde monopolaire à un seul microcâble dont le dispositif de protection atraumatique est porté par le câble de coeur du microcâble de la microsonde, qu'à une sonde multipolaire formée d'un toron d'une pluralité de microcâbles dont le dispositif de protection atraumatique est porté par le câble de coeur d'un microcâble de la microsonde situé en position distale.

Ainsi, avec des microsondes conformes à l'invention, le diamètre réduit du microcâble unique ou en position distale permet d'atteindre des veines de très faible section, inférieure à 1 French, notamment les veines du réseau coronaire profond, sans risque de perforation.

En effet, le risque de perforation du vaisseau par une arête ou un brin métallique du câble de coeur est écarté par la présence du moyen d'enrobage des brins qui assure l'homogénéité de l'extrémité distale du microcâble.

D'autre part, pour résoudre les problèmes liés au risque de perforation par la structure même du microcâble, l'invention propose de transformer l'extrémité distale du microcâble en une extrémité déformable, capable de s'adapter aux géométries variées du réseau coronaire profond, et incompressible.

C'est la combinaison de ces deux caractéristiques - déformation et incompressibilité - qui permet de résoudre dans sa globalité le risque de perforation des vaisseaux coronaires en garantissant une meilleure répartition des forces de pression sur les parois des veines que celle que présenterait la seule sphère métallique rigide d'enrobage des brins.

Le moyen d'enrobage peut notamment avoir la forme d'une sphère de surface homogène, de diamètre compris entre 0,3 et 0,4 mm. Selon un mode de réalisation particulier, le moyen d'enrobage est réalisé par fusion des extrémités distales des brins métalliques élémentaires. Par fusion laser par exemple, on obtient une extrémité en forme de sphère enserrant l'ensemble des brins métalliques.

Afin d'éviter son arrachement, l'embout de protection se prolonge en direction proximale le long du câble de coeur sous forme d'un revêtement.

Dans un mode de réalisation, l'embout de protection est réalisé par dépôt de silicone. Le silicone est une matière élastique biocompatible, communément utilisée dans les applications médicales, et, plus particulièrement, comme composant des sondes de stimulation. Il est bien connu pour ses capacités à supporter de très grandes déformations et est très peu compressible.

Enfin, une variante de réalisation consiste en ce que l'embout de protection comprend un tube de silicone couvrant le moyen d'enrobage et une extrémité distale réalisée à l'extrémité distale du tube de silicone par conformation de colle silicone.

De façon générale, le matériau déformable et incompressible peut être un matériau du groupe formé par : les silicones, les polyuréthannes, les polyéthers, et les copolymères et combinaisons des précédents.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1a est une vue partielle en perspective de l'extrémité distale d'un microcâble d'une microsonde monopolaire portant un dispositif de protection conforme à l'invention.
La Figure 1b est une vue en coupe du câble de coeur du microcâble de la Figure 1a.
La Figure 1c est une vue en coupe d'un brin élémentaire du câble de coeur de la Figure 1 b.
La Figure 2a est une vue partielle en perspective d'une microsonde multipolaire dont l'extrémité distale d'un microcâble porte un dispositif atraumatique de protection conforme à l'invention.
La Figure 2b est une section droite de la microsonde de la Figure 2a.
La Figure 3a est une vue de côté d'un câble de coeur de microcâble muni d'un moyen d'encapsulation de brins élémentaires.
La Figure 3b est une vue en CAO de la Figure 3a.
La Figure 4a est une vue de côté de l'extrémité distale d'un câble de coeur de microcâble muni d'un embout de protection.
La figure 4b est une vue en CAO de la Figure 4a.
La Figure 5a est une vue de côté de l'extrémité distale du câble de coeur de la Figure 4a dont l'embout de protection est déformé.
La figure 5b est une vue en CAO de la Figure 5a.
La Figure 6 est une vue en coupe montrant l'extrémité distale de la Figure 4a stoppée dans une veine du réseau coronaire profond.
La Figure 7 est une vue en coupe montrant l'extrémité distale de la Figure 5a stoppée contre une paroi veineuse du réseau coronaire profond.
La Figure 8 est une vue en coupe d'une variante de réalisation du dispositif atraumatique de protection conforme à l'invention.

On va maintenant décrire des exemples de réalisation de la microsonde de l'invention.

Les microsondes concernées par l'invention sont des microsondes de détection/stimulation destinées à être implantées dans des réseaux veineux, artériels ou lymphatiques. Il peut s'agir aussi bien de microsondes monopolaires comportant un seul microcâble, que de microsondes multipolaires comportant une pluralité de microcâbles.

Dans un contexte plus spécifique, ces microsondes sont destinées à des applications de stimulation cardiaque, notamment de resynchronisation du rythme cardiaque, qui impliquent de pouvoir positionner les électrodes de stimulation dans le réseau coronaire veineux, ce qui suppose de pouvoir atteindre des veines de faible diamètre (et par voie de conséquence de faible épaisseur). C'est pourquoi l'invention prévoit qu'au moins un microcâble des microsondes selon l'invention ait un diamètre hors-tout au plus égal à 1,5 French (0,50 mm).

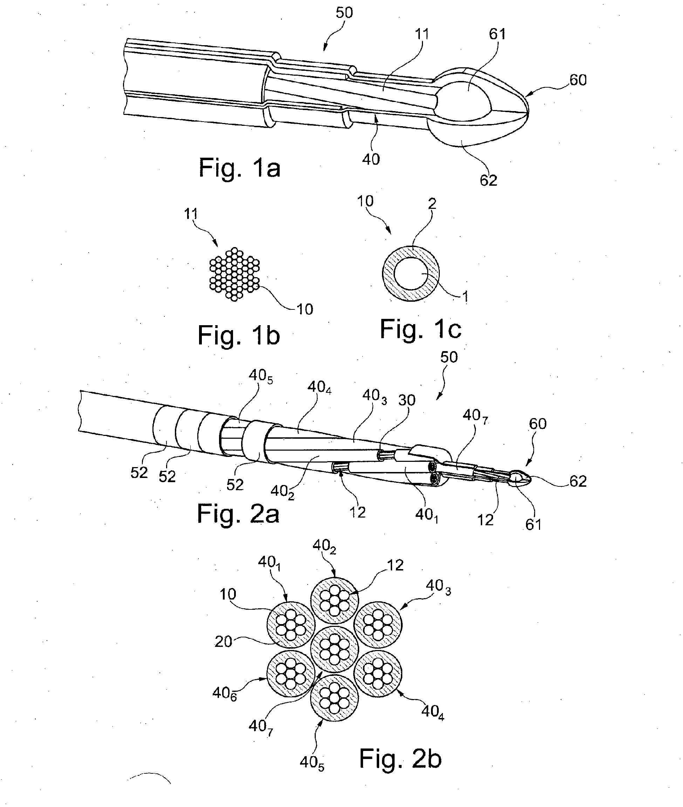

La microsonde 50 montrée sur la Figure 1a est du type monopolaire à un microcâble 40 dont le câble 11 de coeur conducteur est, comme l'indique la Figure 1b, constitué de sept torons de sept brins métalliques élémentaires 10 eux-mêmes assemblés en torons. Le diamètre d'un brin élémentaire est, par exemple, de 0,033 mm. Le diamètre du câble 11 de coeur du microcâble 40 est alors de 0,3 mm.

Il est proposé par l'invention d'utiliser des brins élémentaires 11 du type de celui représenté sur la Figure 1c, c'est-à-dire constitué d'une âme 1 en un matériau structurant comme un acier inox, un alliage de cobalt de la série MP35N, un métal précieux, le titane ou un alliage NiTi, de résistance à la fatigue élevée, le diamètre de 0,033 mm permettant en moyenne d'assurer une résistance à la rupture en fatigue maximale dans les conditions extrêmes de contrainte auxquelles de telles structures peuvent être soumises.

Afin de garantir une visibilité aux rayons X suffisante pour l'implantation de la microsonde, il peut être nécessaire d'introduire le long du brin 10 une quantité minimale de matériau radio-opaque 2 selon une structure composite conciliant résistance à la fatigue du câble et radio-opacité, la plupart des matériaux utilisés pour leur visibilité aux rayons X, à savoir le tantale (Ta), le tungstène (W), l'iridium (Ir), le platine (Pt) et l'or (Au) ne présentant pas en général une grande résistance à la fatigue.

Au surplus, avec les techniques médicales modernes d'imagerie, telles que l'IRM, le faible diamètre donné aux brins est favorable à la dissipation thermique et réduit les effets d'échauffement dus à l'IRM. De plus, l'énergie thermique emmagasinée par les matériaux, déjà limitée en volume, peut être encore diminuée si les brins unitaires sont revêtus d'une couche extérieure en matériau à faible susceptibilité magnétique (la susceptibilité magnétique étant la faculté d'un matériau à s'aimanter sous l'action d'un champ magnétique extérieur).

Les matériaux les plus favorables dans cette application sont ceux dont la susceptibilité magnétique est inférieure à 2000.10⁻¹².m³.mole⁻¹, notamment le tantale (Ta), le titane (Ti), le rhodium (Rh), le molybdène (Mo), le tungstène (W), le palladium (Pd) et l'or (Au).

La microsonde 50' montrée sur la Figure 2a est du type multipolaire et comprend une pluralité de sept microcâbles 40₁, 40₂, 40₃, 40₄, 40₅, 40₆, 40₇ assemblés selon un toron représenté plus spécialement à la Figure 2b en section droite, chaque microcâble constituant pour la microsonde 50' une ligne de conduction reliée à un pôle du générateur.

Comme le montre la Figure 2b, un microcâble comprend un câble 12 de coeur entouré d'une couche 20 d'isolement de manière à assurer l'isolation électrique des microcâbles entre eux.

Dans l'exemple de réalisation de la Figure 2b, chaque câble 12 de coeur est formé par un toron de sept brins élémentaires 10 dont le diamètre est, par exemple, également de 0,033 mm. Le diamètre d'un câble 12 de coeur est alors de 0,1 mm.

Pour réaliser la couche 20 d'isolement, on privilégiera les matériaux à forte inertie chimique comme les polymères fluorés, qui présentent également une très bonne qualité d'isolation. Parmi ces composés, on peut citer en particulier l'ETFE (éthylène tétrafluoroéthylène).

Comme on peut le voir sur la Figure 2a, les couches 20 d'isolement entourant les câbles 12 de coeur des microcâbles présentent au moins une zone dénudée 30 destinée à former pour la microsonde 50' une électrode de détection/stimulation, comme les électrodes 52 de la Figure 2a. Les zones dénudées 30 sont obtenues notamment par la technique d'ablation laser.

Dans cet exemple de réalisation, on peut observer que le microcâble central 40₇ s'étend au-delà des autres microcâbles de la microsonde 50', ceci de manière à pouvoir installer une ou plusieurs électrodes à son extrémité distale. Bien entendu, l'extrémité distale du microcâble 40 de la microsonde 50 de la Figure 1a peut également être munie d'une ou plusieurs électrodes.

On a déjà mentionné plus haut qu'il existe un risque de perforation des veines du réseau coronaire profond dû aux irrégularités pouvant apparaitre aux extrémités distales des microcâbles lors de leur fabrication. En effet, l'utilisation d'une pince coupante pour couper les microcâbles entraine la formation d'arêtes blessantes, de bouts pointus et d'angles vifs. Une alternative est alors l'utilisation d'un tir laser. Dans ce cas, le risque observé est que le bobinage des brins soit endommagé et qu'un ou plusieurs brins s'écartent de l'isodiamètre nominal et deviennent blessants. C'est pourquoi, comme on peut le voir sur les Figures 1a et 2a, les extrémités distales des microcâbles 40 et 40₇, susceptibles d'atteindre des veines du réseau coronaire profond, sont munies d'un dispositif 60 de protection atraumatique. Ce dispositif 60 est essentiellement constitué de deux parties principales, à savoir un moyen 61 d'enrobage des extrémités distales des brins élémentaires du câble 11, 12 de coeur, et un embout 62 de protection en matériau déformable et incompressible, enveloppant le moyen 61 d'enrobage.

Par ses propriétés mécaniques de déformabilité et d'incompressibilité et sa biocompatibilité, le silicone constitue un matériau de choix pour réaliser l'embout 62.

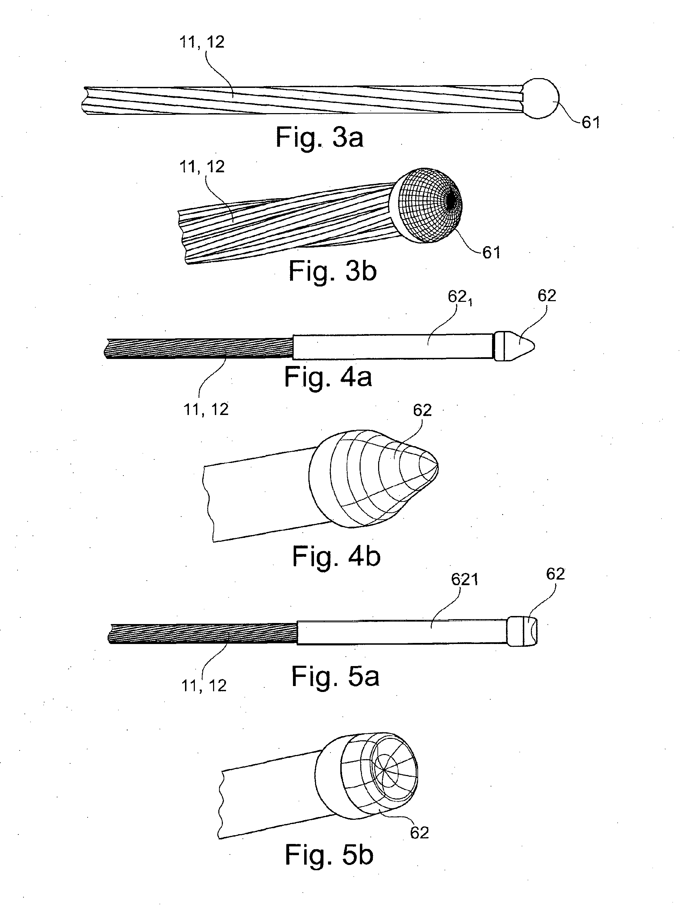

Le moyen 61 d'enrobage a pour but d'éliminer les inconvénients liés aux irrégularités de coupe des microcâbles. Dans l'exemple de réalisation illustré sur les Figures 3a et 3b, le moyen d'enrobage est réalisé par fusion par un tir laser des extrémités distales des brins métalliques élémentaires 10 constituant les câbles 11, 12 de coeur. La transformation de l'extrémité du microcâble en une surface sphérique homogène permet donc de supprimer les risques de perforation dus à des irrégularités dans les câbles 11, 12 de coeur.

L'embout 62 de protection, représenté sur les Figures 4a et 4b avec une extrémité distale conique, a pour objet de remédier aux problèmes liés à la rigidité de la sphère métallique formant le moyen 61 d'enrobage. En effet, cette structure, non déformable, ne permet pas d'adapter l'extrémité distale des microcâbles à la géométrie des vaisseaux coronaires profonds. La déformabilité de l'embout 62 résout cette première difficulté. D'autre part, les pressions appliquées par la sphère d'enrobage sur les parois veineuses, lors de l'insertion de la microsonde par exemple, peuvent être relativement importantes du fait que les surfaces de contact sphère/parois sont faibles. Comme le montrent les Figures 5a et 5b, l'extrémité distale de l'embout 62 est apte à se déformer et s'aplatir axialement en conservant l'aire des surfaces en contact. La déformabilité et l'incompressibilité de l'embout 62 garantissent que les forces exercées par le médecin se répartissent sur une surface maximale et limitent donc la pression sur les parois des veines.

On remarquera que la sphère métallique 61 à l'extrémité du microcâble permet également de maintenir mécaniquement l'embout 62 en silicone. Pour augmenter la tenue à l'arrachement, l'embout 62 de protection se prolonge le long du câble de coeur sous forme d'un revêtement 621. Avantageusement, le silicone est collé sur l'ensemble de la longueur du revêtement 621.

En ce qui concerne les aspects dimensionnels, pour une version monopolaire (avec un unique microcâble tel que le microcâble 40 de la Figure 1a), la sphère 61 peut présenter un diamètre compris entre 0,3 et 0,4 mm. Pour que l'embout 62 puisse passer dans un cathéter implantable de diamètre intérieur 1,6 French (0,53 mm), il convient que l'épaisseur du gainage de silicone soit de l'ordre de 0,1 mm maximum, soit 0,5 mm environ de diamètre hors-tout sur la longueur du câble, et localement 0,6 mm au niveau de la sphère 61, compte tenu de la faculté de déformation locale permise à cet endroit du gainage de silicone et du tube fin du cathéter au passage de l'extrémité distale de la sonde.

Pour une version multipolaire (avec plusieurs microcâbles 40₁ à 40₇ comme illustré sur la Figure 2a), seul le microcâble central 40₇ de 0,1 mm de diamètre supporte une extrémité distale avec la sphère 61 et l'embout 62 en silicone. Dans cette configuration, l'épaisseur du recouvrement peut varier de 0,2 à 0,5 mm maximum, la limitation étant celle du passage dans le cathéter.

Enfin, il faut mentionner la possibilité d'incorporer un stéroïde de type dexaméthasone dans le recouvrement en silicone de l'embout 62, par exemple par mélange de silicone avec de la poudre de dexaméthasone, procédé couramment utilisé dans la fabrication de sondes cardiaques.

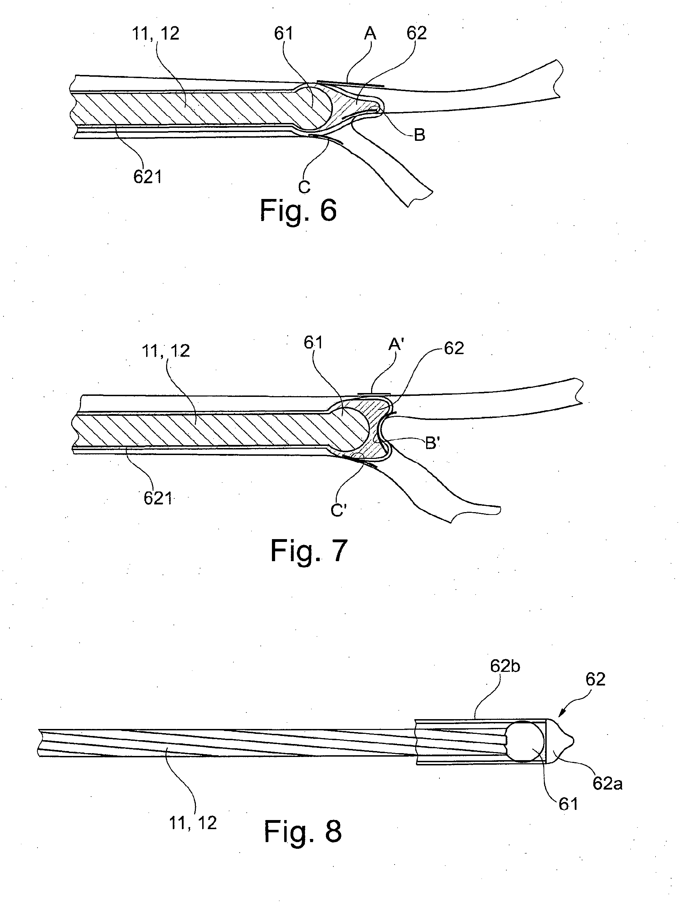

La Figure 6 illustre un premier exemple de situation présentant un risque de perforation potentiel où le microcâble de la microsonde arrive face à une veine de diamètre réduit.

Dans le cas d'un microcâble sans dispositif atraumatique, la paroi veineuse devrait résister seule à la déformation produite par la force de pression exercée par la microsonde. Le risque de perforation est alors dépendant de l'effort appliqué par le médecin et de la résistance des parois veineuses en contact.

Avec le dispositif atraumatique de l'invention, l'embout 62 de protection va se déformer sans être compressé, ce qui contribue à orienter la microsonde dans une veine de diamètre inférieur du réseau coronaire, réaliser le contact avec les parois de la veine et stopper la progression de la microsonde. Dans ce cas, l'aire de la surface de silicone en contact avec les parois veineuses est conservée, et assure une meilleure répartition des pressions sur les parois (les surfaces de veine sollicitées sont indiquées par les références A, B et C).

Dans l'exemple de la Figure 7, l'extrémité de l'embout 62 est bloquée par un contact frontal avec une paroi veineuse réseau coronaire profond. La veine et l'embout 62 se déforment pour absorber la force de pression. L'embout adopte alors une géométrie déformée comme celle montrée sur les Figures 5a et 5b. La déformation du silicone définit des surfaces A', B' et C' de contact dont l'aire totale est bien plus grande que celle correspondant à un microcâble dépourvu du système atraumatique de l'invention. Il en résulte ici encore une meilleure répartition des pressions appliquées aux parois.

La Figure 8 représente une variante avantageuse qui permet de garantir une épaisseur constante de revêtement de silicone le long du microcâble.

Selon cette variante, l'embout 62 de protection comprend un tube 62b de silicone collé sur le câble 11, 12 de coeur et couvrant exactement la sphère métallique 61 d'enrobage. La géométrie déformable 62a en extrémité distale est réalisée à l'extrémité du tube 62b de silicone, par exemple par conformation manuelle de colle silicone.

## Revendications

1. Une microsonde de détection/stimulation destinée à être implantée dans des réseaux veineux, artériels ou lymphatiques,
cette microsonde comportant au moins un microcâble (40 ; 40₇) de diamètre hors-tout au plus égal à 1,5 French (0,50 mm) comprenant un câble de coeur (11 ; 12) électriquement conducteur formé par au moins un toron d'une pluralité de brins métalliques élémentaires (10),
**caractérisée en ce que** le câble de coeur est muni à son extrémité distale d'un dispositif de protection atraumatique comprenant :
- un moyen d'enrobage (61) des extrémités distales des brins élémentaires du câble de coeur ; et
- un embout de protection (62) enveloppant le moyen d'enrobage (61), cet embout de protection présentant une extrémité distale conique en un matériau à la fois déformable et incompressible, apte à se déformer et à s'aplatir axialement en conservant l'aire des surfaces en contact, lors d'une venue en contact de l'extrémité distale de la microsonde avec une paroi d'un vaisseau dans lequel cette microsonde est insérée.

2. La microsonde de la revendication 1, dans laquelle le moyen d'enrobage (61) a la forme d'une sphère de surface homogène.

3. La microsonde de la revendication 2, dans laquelle le diamètre de la sphère est compris entre 0,3 et 0,4 mm.

4. La microsonde de la revendication 1, dans laquelle le moyen d'enrobage (61) est réalisé par fusion des extrémités distales des brins métalliques élémentaires (10).

5. La microsonde de la revendication 1, dans laquelle l'embout de protection (62) se prolonge en direction proximale le long du câble de coeur sous forme d'un revêtement (621).

6. La microsonde de la revendication 1, dans laquelle l'embout de protection (62) est réalisé par dépôt de silicone.

7. La microsonde de la revendication 1, dans laquelle l'embout de protection (62) comprend un tube (62b) de silicone couvrant le moyen d'enrobage (61) et une extrémité distale (62a) réalisée à l'extrémité distale du tube de silicone par conformation de colle silicone.

8. La microsonde de la revendication 1, dans laquelle, la microsonde (50) étant une sonde monopolaire à un microcâble (40), le dispositif de protection atraumatique est porté par le câble de coeur (11) du microcâble de la microsonde.

9. La microsonde de la revendication 1, dans laquelle, la microsonde (50') étant une sonde multipolaire formée d'un toron d'une pluralité de microcâbles (40₁, 40₂, 40₃, 40₄, 40₅, 40₆, 40₇), le dispositif de protection atraumatique est porté par le câble de coeur (12) d'un microcâble (40₇) situé en position distale de la microsonde.

10. La microsonde de la revendication 1, dans laquelle ledit matériau déformable et incompressible est un matériau du groupe formé par : les silicones, les polyuréthannes, les polyéthers, et les copolymères et combinaisons des précédents.

## Patentansprüche

1. Mikrosonde zur Erfassung/Stimulation, die dazu vorgesehen ist, in venöse, arterielle oder lymphatische Netzwerke implantiert zu werden,
wobei diese Mikrosonde mindestens ein Mikrokabel (40; 40₇) mit einem Gesamtdurchmesser von höchstens 1,5 French (0,50 mm) umfasst, das einen elektrisch leitenden Kabelkern (11; 12) aufweist, der aus mindestens einer Litze einer Vielzahl von elementaren metallischen Adern (10) ausgebildet ist,
**dadurch gekennzeichnet, dass** der Kabelkern an seinem distalen Ende mit einer atraumatischen Schutzvorrichtung versehen ist, aufweisend:
- ein Ummantelungsmittel (61) der distalen Enden der elementaren Adern des Kabelkerns; und
- ein schützendes Ader-Endstück (62), mit dem das Ummantelungsmittel (61) umhüllt ist, wobei dieses schützende Ader-Endstück ein konisches distales Ende aus einem Material aufweist, das gleichzeitig verformbar und nicht komprimierbar ist, das in der Lage ist, sich zu verformen und sich axial abzuflachen, und dabei die Stelle der Kontaktoberflächen beizubehalten, wenn das distale Ende der Mikrosonde mit einer Wand eines Gefäßes in Kontakt kommt, in das diese Mikrosonde eingeführt ist.

2. Mikrosonde nach Anspruch 1, wobei das Ummantelungsmittel (61) die Form einer Kugel mit homogener Oberfläche hat.

3. Mikrosonde nach Anspruch 2, wobei der Durchmesser der Kugel zwischen 0,3 und 0,4 mm liegt.

4. Mikrosonde nach Anspruch 1, wobei das Ummantelungsmittel (61) aus der Verschmelzung der distalen Enden der elementaren metallischen Adern (10) hergestellt ist.

5. Mikrosonde nach Anspruch 1, wobei sich das schützende Ader-Endstück (62) in proximaler Richtung entlang des Kabelkerns in Form einer Umhüllung (621) erstreckt.

6. Mikrosonde nach Anspruch 1, wobei das schützende Ader-Endstück (62) aus einer Silikonschicht hergestellt ist.

7. Mikrosonde nach Anspruch 1, wobei das schützende Ader-Endstück (62) eine Silikonhülse (62b), die das Ummantelungsmittel (61) überdeckt, und ein distales Ende (62a) aufweist, das an dem distalen Ende der Silikonhülse durch Formgebung von Silikonklebstoff hergestellt wird.

8. Mikrosonde nach Anspruch 1, wobei die Mikrosonde (50) eine monopolare Sonde mit einem Mikrokabel (40) ist, wobei die atraumatische Schutzvorrichtung an dem Kabelkern (11) des Mikrokabels der Mikrosonde angebracht ist.

9. Mikrosonde nach Anspruch 1, wobei die Mikrosonde (50') eine multipolare Sonde ist, die aus einer Litze einer Vielzahl von Mikrokabeln (40₁, 40₂, 40₃, 40₄, 40₅, 40₆, 40₇) ausgebildet ist, wobei die atraumatische Schutzvorrichtung an dem Kabelkern (12) eines Mikrokabels (40₇) angebracht ist, das an einer distalen Position der Mikrosonde gelegen ist.

10. Mikrosonde nach Anspruch 1, wobei das verformbare und nicht komprimierbare Material ein Material der Gruppe ist, die aus Silikonen, Polyurethanen, Polyethern und Copolymeren und Kombinationen der vorhergehenden besteht.

## Claims

1. A detection/stimulation micro-lead intended to be implanted in venous, arterial or lymphatic systems, this micro-lead comprising at least one micro-cable (40 ; 40₇) of whole diameter at most equal to 1.5 French (0.50 mm) comprising an electrically conductive core cable (11 ; 12) formed by at least one strand of a plurality of elementary metal conductors (10),
**characterized in that** the core cable is provided at its distal end with an atraumatic protection device comprising:
- a means (61) for coating the distal ends of the elementary conductors of the core cable; and
- a protection tip (62) enveloping the coating means (61), this protection tip having a conical distal end made of a both deformable and incompressible material, adapted to be axially deformed and flattened while keeping the surface areas of the surfaces in contact when the distal end of the micro-lead is brought into contact with a wall of a vessel in which this micro-lead is inserted.

2. The micro-lead of claim 1, wherein the coating means (61) has the shape of a sphere of homogeneous surface.

3. The micro-lead of claim 2, wherein the diameter of the sphere is comprised between 0.3 and 0.4 mm.

4. The micro-lead of claim 1, wherein the coating means (61) is made by melting of the distal ends of the elementary metal conductors (10).

5. The micro-lead of claim 1, wherein the protection tip (62) is continued in the proximal direction along the core cable as a coating (621).

6. The micro-lead of claim 1, wherein the protection tip (62) is made by deposition of silicone.

7. The micro-lead of claim 1, wherein the protection tip (62) comprises a silicone tube (62b) covering the coating means (61) and a distal end (62a) made at the distal end of the silicone tube by conformation of silicone adhesive.

8. The micro-lead of claim 1, wherein, the micro-lead (50) being a monopolar lead with one micro-cable (40), the atraumatic protection device is carried by the core cable (11) of the micro-cable of the micro-lead.

9. The micro-lead of claim 1, wherein, the micro-lead (50') being a multipolar lead formed of a strand of a plurality of micro-cables (40₁, 40₂, 40₃, 40₄, 40₅, 40₆, 40₇), the atraumatic protection device is carried by the core cable (12) of a micro-cable (40₇) located at a distal position of the micro-lead.

10. The micro-lead of claim 1, wherein said deformable and incompressible material is a material of the group formed by: silicones, polyurethanes, polyethers, copolymers and combinations thereof.
